(19) Europäisches Patentamt
European Patent Office
Office européen des brevets

(11) Publication number: **0 061 219**
**A1**

(12) # EUROPEAN PATENT APPLICATION

(21) Application number: 82200291.1

(22) Date of filing: 08.03.82

(51) Int. Cl.³: C 07 D 207/46

(30) Priority: 24.03.81 NL 8101443

(43) Date of publication of application: 29.09.82 Bulletin 82/39

(84) Designated Contracting States: BE DE FR GB IT NL

(71) Applicant: Océ-Andeno B.V.
Grubbenvorsterweg 8
NL-5928 NX Venlo(NL)

(72) Inventor: Roy, Peter Daniel
Berkenlaan 47
Velden(NL)

(72) Inventor: Bruggink, Alle
Maricollenweg 3
Grubbenvorst(NL)

(74) Representative: Bleukx, Lucas Lodewijk Maria, Ir. et al,
Océ-Nederland B.V. Patents & Information Dept.
Postbus 101
NL-5900 MA Venlo(NL)

(54) **p-Hydroxyphenylmalonic acid derivatives and process for their preparation.**

(57) The invention relates to isolated para-hydroxyphenylamalonic acid derivatives of formula

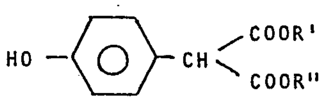

in which R' is an active ester group and R'' is a hydrogen atom or a carboxyl-protective group, as well as to a process for preparing them. Preferably R' is an N-succinimidyl group and R'' is a hydrogen atom or a p-methoxybenzyl group.

EP 0 061 219 A1

# p-Hydroxyphenylmalonic acid derivatives and process for their preparation

This invention relates to p-hydroxyphenylmalonic acid derivatives and to a process for their preparation.

Recently, derivatives of p-hydroxyphenylmalonic acid have roused interest, because they can be used for acylating the amino group of a β-lactam ring (formation of a peptide bond). If such a β-lactam ring forms part of a penicillin or cephalosporin, a particularly useful, pharmaceutically active compound will be formed in that way. In other words: in synthetic antibiotics the p-hydroxyphenylmalonic acid derivative appears to develop as a highly attractive side chain. The formation of a peptide bond between the amino group of the β-lactam ring and the carboxyl function of the desired side chain is possible by various methods.These methods to couple the amino group to the carboxyl function have been derived mainly from the field of peptide chemistry. If a p-hydroxyphenylmalonic acid structure (see formula I, wherein P' = P" = H,A = OH)

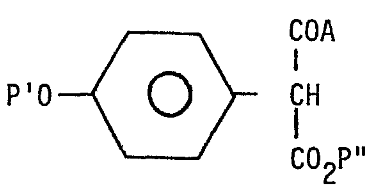

I; (P' and P" = protective groups
A = activating group,)

is used as a carboxyl function in the coupling process, it will generally be necessary for one or both of the functional groups (the phenolic OH and the second carboxyl group) first to be protected (by the group P' and P", respectively) after which still activation (with group A) of the first carboxyl group is required before it is possible

to carry out the coupling reaction with the amino group of the β-lactam ring. Finally, the protective groups will have to be removed to obtain the desired β-lactam product.

Thus, British Patent Specification 1 547 351 describes the coupling of various p-hydroxyphenylmalonic acid derivatives to the C-7 amino group of 1-oxadethiacephalosporin. In all cases there is started from the monoester II as acylating agent

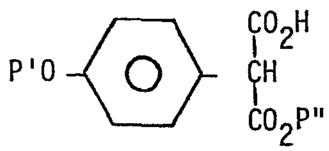

II

wherein either both of the reactive positions have been protected by protective groups P' and P" (see the Examples II: 16,25,26 and 27) or one reactive position has been protected (P' = H; see the Examples II: 7,8,17 and 18). During the coupling process, first the activated form of II is prepared by generating the acid chloride *in situ* or, in one case (see Example II:7), the mixed anhydride, after which the coupling with the amino group follows.

In the Examples where both of the reactive positions have been protected, the yield is approximately 75% or more. If only one reactive position has been protected, the yield may decrease to about 42%.

The British Patent Specification 1 570 582 describes in Example 28 the coupling of p-methoxyphenylmalonic acid to the amino group of a cephalosporin, where activation occurs again *in situ* using a carbodiimide. The yield was about 43%. The same process is also described for p-hydroxyphenylmalonic acid in place of p-methoxyphenylmalonic acid, but as appears from Example 27 the reaction product is then obtained in a much lower and economically uninteresting yield of 25%. From latter Example it is evident that the approach to carry out the coupling reaction with an unprotected, non-activated p-hydroxyphenylmalonic acid is less suitable.

But also the reaction with a p-hydroxyphenylmalonic acid derivative that has been protected and activated shows disadvantages. The preliminary protection of reactive groups, activation of the carboxyl function followed by removal of the reactive groups usually mean as many reaction steps making the process cumbersome, time-absorbing and

expensive.

It is the object of the present invention to provide p-hydroxyphenylmalonic acid derivatives to which the above-mentioned disadvantages do not adhere and which nevertheless give high yields in the coupling reaction with the amino group. As a result, the synthesis of the aforesaid β-lactam antibiotics can be effected in a simpler and cheaper way.

Thus, the invention relates to p-hydroxyphenylmalonic acid derivatives characterized by the formula

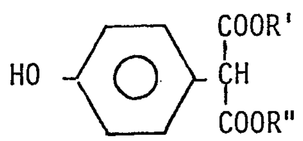

III

wherein

R' is an active ester group derived from N-hydroxysuccinimide, N-hydroxyphthalimide, p- or o-nitrophenol, pentachloro- or pentafluorophenol, chloroacetonitrile or 1-hydroxybenzotriazole, and

R" is a hydrogen atom or a carboxyl- protective group derived from p-methoxybenzyl alcohol, benzyl alcohol, benzhydryl alcohol or t-butyl alcohol.

How the term "active ester group" has to be interpreted will generally be obvious to those skilled in the art. For the sake of completeness it may be remarked that information on this term is to be found in chapter 3: "Active esters in peptide synthesis" by M.Bodanszky in "The Peptides, Analysis, Synthesis, Biology", Volume 1, edited by E.Gross and J.Meienhofer, Academic Press, New York/London 1979.

Thus, the present invention starts from a p-hydroxyphenylmalonic acid derivative, wherein:

(1) the phenolic hydroxyl group is unprotected,

(2) R' is a representative of the active ester groups conventional in peptide chemistry;
R' preferably being an N-succinimidyl group,

(3) R" is a hydrogen atom or one of the conventional carboxyl-protective groups, which group leaves readily during the coupling reaction. Examples of readily leaving carboxyl- protective groups are to be found in "The Peptides", Volume I: "Methods of Peptide Synthesis",

Academic Press, New York 1965, by E.Schröder and K.Lübke.

Preferred is a p-hydroxyphenylmalonic acid derivative according to formula III, wherein R' = N-succinimidyl and R" = p-methoxybenzyl (see Formula IV)

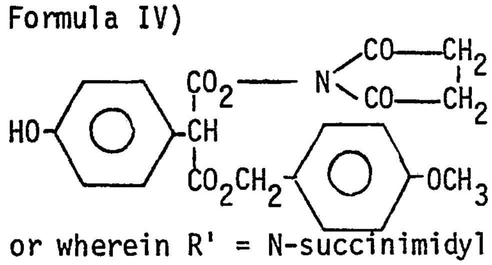

IV

or wherein R' = N-succinimidyl and R" = H (see Formula V)

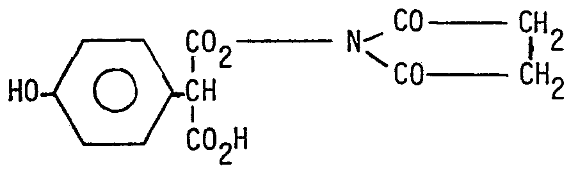

V.

No protective groups are present in the compound according to Formula V, so that also no protective groups have to be removed later on. True, a protective group for the carboxyl-function is present in the compound according to Formula IV, but this need not to be removed either later on in a separate step, because it can be cleaved in a simple way during the coupling process, e.g. by working up the reaction mixture in an acidic medium. So, in both cases the β-lactam ring need not to be subjected to additional chemical operations for removing protective groups, so that the final product is not contaminated from cleaved protective groups nor from chemicals required in the cleaving process. Thanks to the succinimidyl group the carboxyl function in both derivatives has been sufficiently activated to be able to acylate an amino group.

There is a marked difference between the activated malonic acid derivatives according to the invention and those known from the British patent specifications discussed before. The former are isolated products, which as such can be transported, stored and caused to react. The latter are generated *i n s i t u*, for which purpose an extra operation is required which makes the process as a whole more complex and costly, so less attractive.

The invention further relates to a process for preparing p-hydroxyphenylmalonic acid derivatives according to the invention, which process is characterized in that a compound of the formula

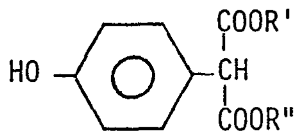

III

wherein R' and R" are each as defined above, is prepared by:

(a) introduction of R" (R" not being H) by a conventional method for esterifying p-hydroxyphenylacetic acid by means of one of the alcohols mentioned above,

(b) carboxylation of the α-C atom of the p-hydroxyphenyl acetate obtained under (a),

(c) introduction of R' by means of a condensation reaction which is known in the art,

(d) followed by treatment with an acid, only in those cases where a derivative with R" = H is desired.

To obtain the preferential compound of formula IV the characteristic reaction steps are:

(e) esterification of p-hydroxyphenylacetic acid by a conventional method using p-methoxybenzyl alcohol to p-methoxybenzyl p-hydroxyphenyl acetate,

(f) carboxylation of the resulting product at the α-C atom, and

(g) causing the product formed under (f) to condensate with N-hydroxysuccinimide under the influence of N,N'-dicyclohexylcarbodiimide.

To obtain the preferential compound V the reaction product obtained under (g) is further treated with an acid.

The following Examples may serve to illustrate the invention. The first examples describe the preparation of a p-hydroxyphenylmalonic acid derivative according to the invention, the last one the coupling of such a derivative with an amino group of a β-lactam ring, which ring forms part of a penicillin. From that last Example it also appears that coupling with an excess of p-hydroxyphenylmalonic acid derivative according to the invention, followed by a working up process, using 3-N,N-dimethylaminopropylamine, may increase the yield of coupling product considerably. For with the diamine the excess of the derivative in question forms a basic amide, which can be removed by washing with diluted acid.

In the Examples some notorious abbreviations being generally adopted are used, the meaning of which is disclosed below, for the sake of completeness:

t.l.c. = thin-layer chromatography

p.m.r. = proton magnetic resonance

TMS = tetramethylsilane

Example 1: Preparation of p-methoxybenzyl, N-succinimidyl diester of p-hydroxyphenylmalonic acid (Formula IV)

This compound was prepared in three stages:

(1) Preparation of p-methoxybenzyl p-hydroxyphenyl acetate

A solution of 304.3 g (=2M) of p-hydroxyphenylacetic acid in 1,200 ml of methanol and 5 ml of concentrated sulphuric acid was refluxed for one hour and then neutralised with 10 ml of triethylamine. After evaporating to dryness (50°C, waterbath) under reduced pressure, the methyl ester of p-hydroxyphenylacetic acid resulted as residue in a yield of 333 g = 100%. Its transesterification occurred by treatment with an excess (1,275 g = 9.2 M) of p-methoxybenzyl alcohol, 110 g (= 2 M) of $NaOCH_3$ and 740 ml of toluene under reflux and nitrogen atmosphere and using a Dean Stark separator.

During the transesterification reaction taking 2½ hour, 850 ml of toluene were added to replace the toluene/methanol mixture being distilled over and tapped off.

The pH was adjusted to 5 using 144 ml of glacial acetic acid and 400 ml of water were added. The organic fraction was isolated and evaporated to dryness in order to remove the toluene (water-pump) and the excess of p-methoxybenzyl alcohol ( 1 mm pressure, bath temperature 150°C). A brown oil (512 g = 100% yield) which slowly crystallized on standing, was left.

Recrystallisation from a toluene/hexane mixture afforded a colourless solid (melting point 79-81°C) which at Rf = 0.60 appeared as one spot on a t.l.c. plate (silica gel; dichloroethane: acetone: formic acid, 18:2:2).

The p.m.r. spectrum confirmed the structure.

(2) Preparation of mono-p-methoxybenzyl p-hydroxyphenyl malonate

At room temperature,with stirring, 1.16 g of 80% (= 0.039 M) NaH were dosed to a solution of 10 g (=0.039 M) of the p-methoxybenzyl p-hydroxyphenyl acetate, obtained above, in 100 ml dry tetrahydrofuran. The reaction mixture was stirred for 3 hours to obtain a solution of the Na-salt of the title compound.

In a separate flask a solution of 7.9 g (= 0.078 M) of diisopropylamine in 50 ml of dry tetrahydrofuran was prepared. To this solution 33 ml of 15% (= 0.078 M) butyllithium in hexane were dosed at -5$^{o}$C to form lithium diisopropylamide.

After stirring for 30 minutes, the solution was cooled to -20$^{o}$C whereupon the Na-phenolate solution made before was added drop by drop for a period of 20 minutes. After further stirring the reaction mixture at -10$^{o}$C for 30 minutes, dry carbon dioxide gas was passed through it for 30 minutes. The reaction mixture was then poured out onto a mixture of ice/excess of 1 N hydrochloric acid and extracted with ether.

The product was purified by extracting it from the ether into an 0.1 N solution of sodium bicarbonate, which was acidified again in the presence of ether. The ether layer was separated and dried. The ether was evaporated under reduced pressure, leaving a solid (in a yield of 7.8 g = 66%) which at Rf = 0.41 appeared as one spot on a t.l.c. plate (silica gel; dichloroethane: acetone: formic acid, 18:2:2). The melting point was 92 - 3$^{o}$C.

The p.m.r. was in accordance with the structure.

(3) Preparation of p-methoxybenzyl N-succinimidyl diester of p-hydroxyphenylmalonic acid

A solution of 9.0 g (= 0.03 M) of the p-methoxybenzyl p-hydroxyphenyl malonate obtained under (2) in 30 ml of ethyl acetate was added to a solution of 3.45 g (= 0.03 M) of N-hydroxysuccinimide in 120 ml of ethyl acetate. After cooling the resulting solution to 0$^{o}$C, a solution of 6.79 g (= 0.033 M) of dicyclohexylcarbodiimide in 15 ml of ethyl acetate was added. The reaction mixture was first stirred for one hour at 0$^{o}$C, subsequently for 3 hours at room temperature and then filtered.

The filtrate was evaporated to dryness under reduced pressure and the residue was crystallised from a mixture of ethyl acetate and petroleum ether ( 60$^{o}$ - 80$^{o}$C). The result was 8.65 g (=73% yield) of a colourless solid having a melting point of 133 - 4$^{o}$C (decomposition) which at Rf = 0.45 appeared as one spot on a t.l.c. plate (silica gel; dichloroethane: acetone: formic acid: 18:2:2).

The p.m.r. spectrum, recorded in a 10% acetone-$d_6$ solution with TMS as a reference material, showed peaks at δ 2.80 (succinimidyl $CH_2$,s,4H), 3.75 ($OCH_3$,s,3H), 5.10 (anisyl $CH_2$ and α-CH,s,3H), 6.80 and 7.30 (aromatic protons, 8 H) and 8.48 (phenolic OH,s,1H)

Example 2: Preparation of mono-N-succinimidyl p-hydroxyphenyl malonate (Formula V)

A solution of 3.0 g of p-methoxybenzyl N-succinimidyl diester of p-hydroxyphenylmalonic acid in 52 ml of a 1:2:4 mixture of trifluoroacetic acid: methoxybenzene: methylene chloride was allowed to stand at room temperature for 18 hours. 1.15 g (= 55%) of colourless crystals of the title compound deposited. This product was filtered off, washed with ether and dried *in vacuo*. The melting point was 134 - 5°C (decomposition). On a t.l.c. plate (silica gel; dichloroethane: acetone, formic acid, 18:2:2) the product appeared as one spot (Rf = 0.23).

The p.m.r. spectrum, recorded in a 5% acetone -$d_6$ solution with TMS as a reference material, showed peaks at δ 2.85 (succinimidyl $CH_2$,s, 4H), 5.07 ( α-CH,s,1H), 6.87 and 7.39 (aromatic protons, 4H) and 6.60 (phenolic OH and α-COOH,s, broad peak).

Example 3: Acylation of benzyl 6-aminopenicillinate with p-methoxybenzyl N-succinimidyl diester of p-hydroxyphenylmalonic acid

a) without excess diester

To a solution of 1.53 g (= 5mM) of benzyl 6-aminopenicillinate in 15 ml of acetonitrile was added a solution of 2.06 g (= 5mM) of p-methoxybenzyl N-succinimidyl diester of p-hydroxyphenylmalonic acid in 10 ml of acetonitrile. To the resulting mixture was added 0.7 ml of N-methylmorpholine as a catalyst. After stirring for 3 hours at room temperature, 25 ml of ethyl acetate was added and then the reaction mixture was washed successively with 0.1 N hydrochloric acid, water, 5% solution of sodium bicarbonate and, finally, water.

The organic layer was separated, dried (magnesium sulphate) and evaporated to dryness, leaving a colourless, brittle, foamy material (2.40 g = 81.6% yield).

The p.m.r. spectrum was in accordance with the structure and identical to that of a reference sample prepared by a different route, namely,

by a carbodiimide coupling of benzyl 6-aminopenicillinate with the mono-p-methoxybenzyl p-hydroxyphenyl malonate obtained in Example 1(2).

b) with excess diester

To a solution of 1.53 g (= 5mM) of benzyl 6-amino-penicillinate in 15 ml of acetonitrile was added a solution of 3.09 g (= 7.5 mM) of p-methoxybenzyl N-succinimidyl diester of p-hydroxyphenylmalonic acid in 45 ml of acetonitrile. To the resulting mixture was added 0.7 ml of N-methylmorpholine as a catalyst. After stirring for 3 hours at room temperature, 0.31 ml (= 2.5 mM) of 3-dimethylaminopropylamine was added and the reaction mixture was further stirred for 2 hours. After adding 45 ml of ethyl acetate, the reaction mixture was worked up as described under 3 a). Yield 2.65 g = 90.1%.

The p.m.r. spectrum was in accordance with the structure.

-----------------------

CLAIMS

1. Para-hydroxyphenylmalonic acid derivatives characterized by the formula

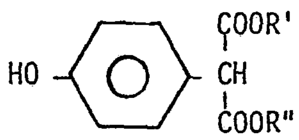

wherein

R' = an active ester group derived from N-hydroxysuccinimide, N-hydroxyphthalimide, p- or o-nitrophenol, pentachloro- or pentafluorophenol, chloroacetonitrile or 1-hydroxybenzotriazole, and

R" = a hydrogen atom or a carboxyl-protective group derived from p-methoxybenzyl alcohol, benzyl alcohol, benzhydryl alcohol or t-butyl alcohol.

2. A compound according to claim 1, characterized in that R' = an N-succinimidyl group.

3. A compound according to claim 2, characterized in that R" = a hydrogen atom or a p-methoxybenzyl group.

4. A process for preparing para-hydroxyphenylmalonic acid derivatives, characterized in that compounds of the formula

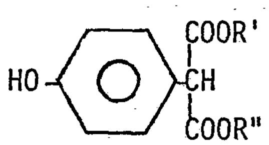

wherein

R' and R" are each as defined in claim 1, are prepared by:

(a) introduction of R" (R" not being H) by a conventional method for esterifying p-hydroxyphenylacetic acid by means of one of the alcohols mentioned in claim 1,

(b) carboxylation of the α-C atom of the p-hydroxyphenyl acetate obtained under (a),

(c) introduction of R' by means of a condensation reaction which is known in the art,

(d) followed by treatment with an acid, only in those cases where a derivative with R" = H is desired.

5. A process according to claim 4, characterized in that a compound of the formula

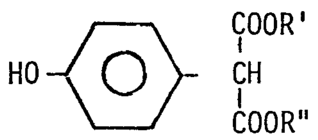

wherein

R' = N-succinimidyl and R" = p-methoxybenzyl, is prepared by:

(a) esterification of p-hydroxyphenylacetic acid by a conventional method using p-methoxybenzyl alcohol to p-methoxybenzyl p-hydroxyphenyl acetate,

(b) carboxylation of the resulting product at the α-C atom, and

(c) causing the product mentioned under (b) to condensate with N-hydroxysuccinimide under the influence of N,N'-dicyclohexycarbodiimide.

6. A process according to claim 5, characterized in that the resulting compound is subjected to a treatment with acid to obtain a derivative wherein R' is an N-succinimidyl group and R" is a hydrogen atom.

----------------

European Patent Office

# EUROPEAN SEARCH REPORT

0061219 Application number

EP 82 20 0291

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | CHEMICAL ABSTRACTS, vol. 92, no. 5, 4th February 1980, page 750, no. 41594q, Columbus Ohio (USA); & JPA 79106447 (SHIONOGI AND CO. LTD.) (21-08-1979) *Abstract* --- | 1,4 | C 07 D 207/46 |
| A | BE-A- 871 213 (SHIONOGI AND CO. LTD.) *Page 97, claim 1* ----- | 1,4 | |
| | | | TECHNICAL FIELDS SEARCHED (Int. Cl. 3) |
| | | | C 07 D 207/00 |

The present search report has been drawn up for all claims

| Place of search | Date of completion of the search | Examiner |
|---|---|---|
| THE HAGUE | 13-05-1982 | MAISONNEUVE J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document